# EUROPEAN PATENT APPLICATION

(11) **EP 3 964 223 A2**
(43) Date of publication of application: **09.03.2022**
(21) Application number: 20799504.4
(22) Date of filing: 29.04.2020
(51) Int. Cl.: A61K 38/00, A61P 17/14, A61K 8/64, A61Q 7/00

(54) **COMPOSITION FOR PREVENTION OR TREATMENT OF HAIR LOSS INCLUDING HAPLN1**

(30) Priority: 30.04.2019 KR 20190050698; 28.04.2020 KR 20200051429
(71) Applicant: HAPLNSCIENCE INC., Gyeonggi-do 13494 (KR)
(72) Inventor: KIM, Dae Kyong, Seongnam-si Gyeonggi-do 13547 (KR); HA, Hae Chan, Seoul 05817 (KR); JANG, Ji Min, Seoul 07435 (KR); SHIN, In Chul, Seoul 04741 (KR); BACK, Moon Jung, Seoul 08750 (KR); ZHOU, Dan, Seoul 06979 (KR)
(74) Representative: Mewburn Ellis LLP
(86) International application number: PCT/KR2020/005703
(87) International publication number: WO 2020/222538

(57) **Abstract**

The present invention relates to a pharmaceutical composition for preventing or treating hair loss, comprising hyaluronan and proteoglycan link protein 1 (HAPLN1) as an active ingredient. When administered ,the HAPLN1 protein of the present invention causes hair growth by promoting the proliferation of hair germinal matrix cells through a Ras-ERK1/2 signaling pathway activated by a TGF-β protein.

## Description

### TECHNICAL FIELD

The present invention relates to a pharmaceutical composition for preventing or treating hair loss, comprising hyaluronan and proteoglycan link protein 1 (HAPLN1) as an active ingredient. The HAPLN1 protein of the present invention makes hair grow by promoting proliferation of hair germinal matrix cells through a ERK1/2 signaling pathway (that is, a non-canonical signaling pathway) activated by a TGF-β protein when administered.

### BACKGROUND ART

Globally, about 35 million males and 25 million females are suffering from hair loss, and the number of hair loss patients has been increasing each year. Hair has various functions including appearance, heat insulation, scalp protection, and friction dampening. Among them, hair is particularly directly related to self-esteem and sociality, and thus many people are very interested in hair care for aesthetic reasons.

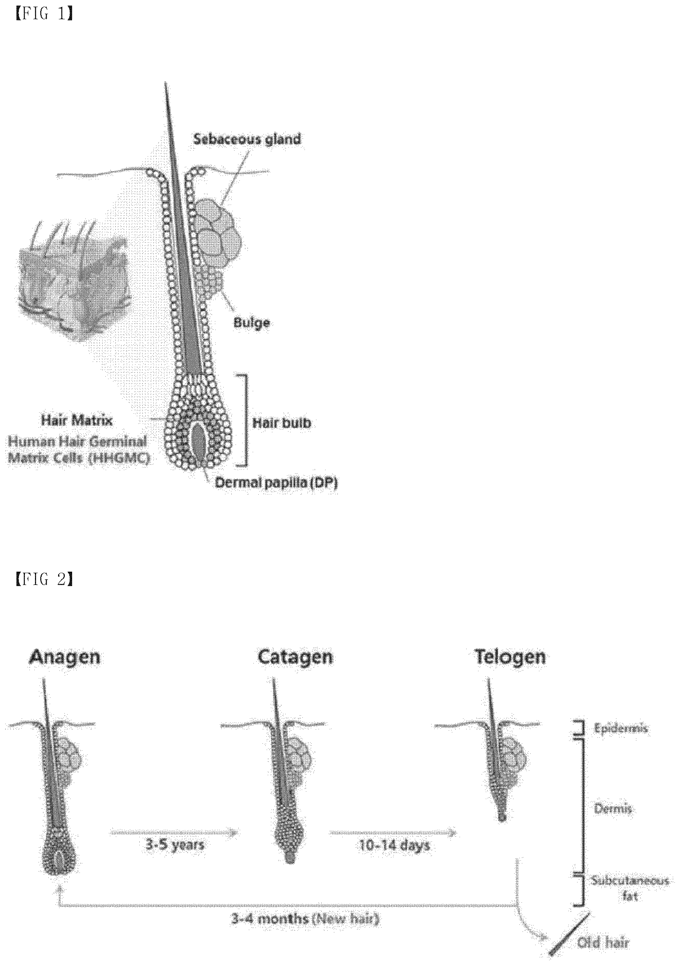

Human hair is an aggregate of about 100,000 individual hairs, each of which is produced by a hair follicle. The hair follicle serves as a reservoir of stem cells that can generate all the cell lines needed to rebuild the follicle itself, the epithelium, and the sebaceous gland. The hair follicle is composed of dermal papilla cells, hair germinal matrix cells, outer and inner walls of hair follicle cells, and a bulge (FIG. 1).

Hair repeats the hair growth cycle of anagen, catagen, and telogen (FIG. 2). The anagen usually lasts for 3-5 years, and is a stage in which hair grows by proliferation and keratinization of keratinocytes of the hair germinal matrix cells as dermal papilla cells and hair germinal matrix cells develop. The catagen lasts 10-14 days, and is a stage in which the hair follicle shrinks as apoptosis of the hair follicle cells occurs. The telogen is maintained for about 3-4 months, and is a stage in which hair generation is stopped, and new cells are supplied to the entire hair follicle to prepare for the next anagen.

Signaling pathways involved in the hair growth cycle include signaling pathways involving Wnt, Shh, JAK, TGFβ/BMP, Testosterone, etc. Among them, the Wnt, Shh, and JAK signaling pathways are activated at the end of the telogen to promote entry into the anagen.

As such, human hair always maintains a constant number of hairs because it has a constant hair growth cycle. However, when hair loss progresses, the papilla present in the hair root becomes smaller, and when the dermal papilla becomes smaller, the thickness of the hair becomes thinner, and, at the same time, the hair period becomes shorter and the newly grown hair becomes thinner. Therefore, when hair loss progresses, the hair strands of the hair becomes fluffy, and the hair cycle becomes much shorter and hair falls out after it has grown a little.

Hair loss can be largely divided into four types: male-pattern hair loss, female-pattern hair loss, alopecia areata, and telogen effluvium. Male pattern hair loss is largely caused by genetic causes and is related to 5α-reductase. 5α-reductase converts the male hormone testosterone into 5α-dihydrotestosterone (DHT), which reduces hair follicles, causing hair loss. The main cause of female pattern hair loss is unbalanced secretion of hormones due to childbirth or menopause. In addition, various habits and environments, including mental stress in social life, exposure to air pollution, consumption of processed foods, severe illness due to high fever, nutritional imbalance, use of anticancer drugs and antithyroid drugs, administration of oral contraceptives, shampoo, strong ultraviolet rays or sweat during exercise, dietary habits , psychological pressure, and so on, are also known as main causes of hair loss.

Currently, the most frequently used hair loss treatments in Korea include finasteride (product name: Propecia^{®}), dutasteride (product name: Avodart^{®}), and minoxidil (product name: Myoxydil^{®} or Rogaine^{®}). Finasteride and Dutasteride are 5α-reductase inhibitors that inhibit the conversion of testosterone to 5α-dihydrotestosterone (DHT). However, these products exhibit side effects such as decreased libido, impotence, and loss of driving and performance capabilities. Meanwhile, Minoxidil, the mechanism of which has not yet been completely elucidated, is a potassium channel opener that hyperpolarizes cell membranes, but is believed to enhance the health of hair follicles by increasing the supply of oxygen, blood, nutrients, etc. to the hair follicles through vasodilation and opening of potassium channels. However, this product also shows side effects, such as itching, erythema, skin irritation, and eye irritation at a portion on which the product is applied, and unwanted hair growth is also observed on body parts other than the head. Since the side effects of these existing products amplify patients' anxiety and, in severe cases, may lead to refusal of administration, constant efforts are being made to develop drugs having fewer side effects.

Hyaluronan and proteoglycan link protein 1 (HAPLN1) is an extracellular matrix protein found in cartilage. This protein plays a role in stabilizing the aggregates of hyaluronic acid and proteoglycans, and is reported to be involved in the binding of cells.

As a recently reported study, U.S. Patent Publication No. 2012/0128632 presents a method for identifying trichogenic dermal cells, such as dermal papilla cells and dermal sheath cells, which can induce hair follicle formation, and proposes HAPLN1 as one of the biomarkers that can be used to detect and identify trichogenic dermal cells. In addition, U.S. Patent Registration No. 8,334,136 lists about 20 genes involved in cell adhesion in dermal papilla cells and mentions the possibility of promoting hair follicle formation by maintaining or increasing the expression of the genes. However, among the 20 genes, HAPLN1 is disclosed in the patent, and the results confirming whether the expression of these genes actually promotes hair follicle formation are not disclosed at all.

The above documents merely mention HAPLN1 as one of the biomarkers for identifying dermal papilla cells or dermal sheath cells or distinguishing them from other cells, and does not mention at all that directly administering the HAPLN1 protein as an active ingredient brings about the effect of preventing or treating hair loss. Furthermore, no research has been conducted to date on the points that among many pathways involved in the hair growth cycle, activation of the ERK1/2 signaling pathway, which is activated by TGF-β protein, is effective in treating hair loss, and, in particular, HAPLN1 acts on germinal matrix cells to activate the above pathway.

### DESCRIPTION OF EMBODIMENTS

### TECHNICAL PROBLEM

An object of the present invention is to provide a pharmaceutical composition for preventing or treating hair loss, which has reduced side effects while having excellent effects of hair loss treatment, compared to conventional hair loss treatments that are accompanied by serious side effects.

Another object of the present invention is to provide a pharmaceutical composition which exhibits an effect of preventing or treating hair loss through the action mechanism different from that used in conventional hair loss treatments.

Another object of the present invention is to provide a pharmaceutical composition for growing hair by promoting the proliferation of hair germinal matrix cells. Hair repeats the cycle of anagen, catagen, and telogen, among which the anagen is a stage in which the germinal matrix cells actively differentiate to generate hair, and the thickness and length of the hair are determined at this stage. Since the anagen is the most essential stage in the treatment of hair loss, it is important to develop a therapeutic agent for promoting the initiation of the anagen of hair follicles or helping proliferation of hair germinal matrix cells during the anagen.

### SOLUTION TO PROBLEM

The present invention provides a pharmaceutical composition for preventing or treating hair loss, comprising HAPLN1 as an active ingredient.

The present invention also provides a cosmetic composition for preventing or improving hair loss, comprising HAPLN1 as an active ingredient.

In one embodiment, the HAPLN1 activates a non-canonical signaling pathway, and the non-canonical signaling pathway is an ERK1/2 signaling pathway activated with a TGF-β protein. Accordingly, the HAPLN1 of the present invention promotes the proliferation of hair germinal matrix cells to enable hair growth.

In one embodiment, the pharmaceutical composition of the present invention may be for the prevention or treatment of male-pattern hair loss, female-pattern hair loss, circular hair loss, or telogen hair loss. In addition, the hair loss may be reduced expression of the HAPLN1 protein in hair germinal matrix cells.

In one embodiment, the pharmaceutical composition of the present invention is used alone or in combination with other therapeutic agents.

### ADVANTAGEOUS EFFECTS OF DISCLOSURE

The pharmaceutical composition of the present invention comprises, as an active ingredient, HAPLN1, which is an intracellular protein constituting the extracellular matrix, and thus has reduced side effects, compared to conventional therapeutic agents of hair loss.

In addition, the HAPLN1 contained in the pharmaceutical composition of the present invention as an active ingredient activates an ERK1/2 signaling pathway activated by a TGF-β protein, that is, a non-canonical signaling pathway, thereby enabling hair growth through proliferation of hair germinal matrix cells. Such action mechanism is completely different from that used in conventional therapeutic agents of hair loss, and has never been known to date. Accordingly, HAPLN1 can be used as a new concept of hair loss treatment, and can suggest a breakthrough strategy and new direction in the research into hair loss treatment in the future.

### BRIEF DESCRIPTION OF DRAWINGS

FIG. 1 is a schematic diagram showing the structure of a hair follicle.
FIG. 2 is a schematic diagram showing a hair growth cycle.
FIG. 3 is a schematic diagram showing the mechanism by which HAPLN1 promotes proliferation of hair germinal matrix cells and hair growth.
FIGS. 4 and 5 show the expression levels of HAPLN1 protein and HAPLN1 mRNA in each of anagen, catagen, and telogen in the hair growth cycle.
FIG. 6 shows results confirming the increase in TβRII protein in human hair germinal matrix cells by HAPLN1.
FIG. 7 shows results confirming the increase in TβRII protein in human hair germinal matrix cells by HAPLN1 and/or HA.
FIG. 8 shows results confirming the effect of endogenous HAPLN1 deficiency on the TβRII protein concentration in human hair germinal matrix cells.
FIG. 9 shows results confirming the effect of exogenous HAPLN1 and/or HA on the TβRII protein concentration in human hair germinal matrix cells deficient in endogenous HAPLN1.
FIG. 10 shows results confirming the effect of exogenous HAPLN1 on the concentration of TβRII and HAS2 proteins in human hair germinal matrix cells deficient in endogenous HA.
FIG. 11 shows results confirming the effect of CD44 deficiency on the TβRII protein concentration in human hair germinal matrix cells.
FIG. 12 shows results confirming the effect(s) of HAPLN1 and/or HA on the concentration of TβRII protein in human germinal matrix cells deficient in CD44.
FIG. 13 shows results confirming the effects of HAPLN1 and/or HA on the cell membrane TβRII protein concentration.
FIGS. 14 to 16 show results confirming the effects of HAPLN1 and/or HA on pERK1/2, p-Smad2, p-MEK1/2, and p-c-Raf in human hair germinal matrix cells.
FIG. 17 shows results confirming that cell proliferation was promoted in the group treated with HAPLN1 and/or HA in the presence of TGF-β2.
FIG. 18 shows results f confirming the expression of HAPLN1 protein and TβRII protein in each hair growth cycle.
FIG. 19 shows an experimental schedule and results confirming the effect of intraperitoneal systemic administration of HAPLN1 on the hair growth of mice.
FIG. 20 shows an experimental schedule and results thereof confirming the effect of intraperitoneal systemic administration of HAPLN1 siRNA on the hair growth of mice.
FIG. 21 shows results confirming cell proliferation when human dermal papilla cells were treated with HAPLN1, CX3CL1, or CDON protein, or minoxidil.
FIG. 22 shows results confirming cell proliferation when human germinal matrix cells were treated with HAPLN1, CX3CL1 or CDON protein.

### BEST MODE

Hereinafter, embodiments and examples of the present invention will be described in detail with reference to the accompanying drawings so that those of ordinary skill in the art may easily implement the present disclosure. However, the present application may be implemented in various forms and is not limited to the embodiments and examples described herein.

Throughout the specification of the present application, when a part "comprises" a certain component, it means that other components may be further included rather than excluding other components unless specifically stated to the contrary.

The present invention relates to a pharmaceutical composition for preventing or treating hair loss, comprising HAPLN1 as an active ingredient. In one embodiment, the present invention provides a method for preventing or treating hair loss in a subject, the method comprising administering an effective amount of HAPLN1 protein to the subject in need thereof. In another embodiment, the present invention provides the use of HAPLN1 protein for preventing or treating hair loss.

HAPLN1 is a protein present in the body, and has been used as a biomarker for identifying specific cells or distinguishing same from other cells in conventional studies related to hair loss treatment. When the HAPLN1 protein of the present invention is directly used as an active ingredient, it exhibits excellent effects of preventing or treating hair loss while having relatively reduced side effects, compared to conventional hair loss therapeutic agents accompanied by serious side effects.

In particular, when the HAPLN1 protein of the present invention is used directly as an active ingredient, hair growth is promoted in a completely different way from the existing hair loss treatment. Specifically, the HAPLN1 protein of the present invention activates a non-canonical signaling pathway, and in this case, the non-canonical signaling pathway is an ERK1/2 signaling pathway that is activated with a TGF-β protein. Through the signaling pathway, HAPLN1 promotes proliferation of hair germinal matrix cells and allows hair growth, thereby exhibiting an effect of preventing or treating hair loss.

The activation of the non-canonical signaling pathway of the HAPLN1 protein, which is identified for the first time in the present invention, will now be described in detail.

With regard to TGF-β signaling mechanism, when a hair germinal matrix cell is stimulated by TGF-β, TGF-β binds to the TGF-β receptor 2 (TβRII) of the hair germinal matrix cell. Thereafter, TβRII binds to TGF-β receptor 1 (TβRI) to form a TβP complex. The TβP complex enters the cell by endocytosis, and the cell cycle is arrested through Smad2/3 signaling during clathrin-dependent endocytosis. This pathway is a canonical signaling pathway. However, when caveolin-1 induces endocytosis, TβRI and TβRII are finally degraded through Smad7 signaling. In this process, the TGF-β signaling pathway is activated, and apart from the canonical Smad pathway, there is Ras-ERK1/2 mechanism of a non-canonical pathway, such as Ras-ERK1. Activation of the Ras-ERK1/2 signaling leads to cell proliferation.

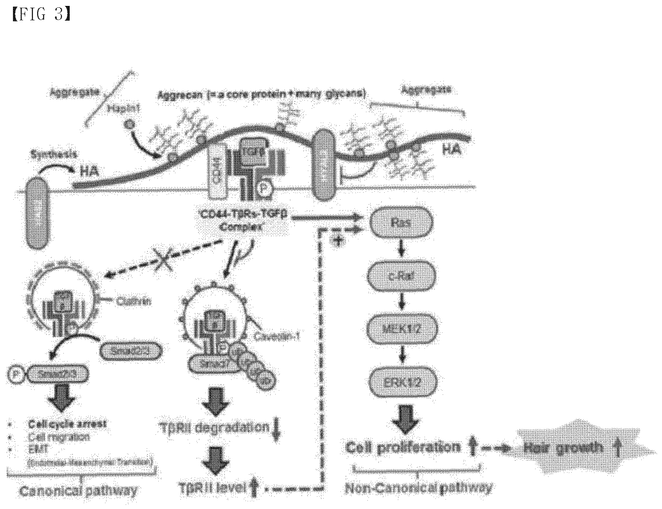

In the present invention, it was revealed for the first time that HAPLN1 activates the Ras-ERK1/2 pathway, that is, a non-canonical pathway in the TGF-β signaling mechanism (FIG. 3). Specific mechanism will now be described. Hyaluronic acid (HA) is produced by hyaluronic acid synthase 2 (HAS2) in cells. HA binds to the cell membrane's CD44 (receptor of HA), and CD44 binds to TβR. That is, HA is indirectly bound to TβR. To induce endocytosis of HA, the HA is essentially decomposed by hyaluronidase (HYAL2). HAPLN1 induces stabilization of HA by linking HA with proteoglycans. HAPLN1 inhibits HYAL2 from decomposing HA by allowing HA to be tightly surrounded by proteoglycans, or inhibits HA from being decomposed by a reactive oxygen species (ROS). As a result, HAPLN1 inhibits the inclusion of TβR, prevents Smad2/3 mechanism activity and degradation of TβR, and increases cell membrane TβRII. Accordingly, the non-canonical signaling pathway, that is, the Ras-ERK1/2 mechanism, is activated by HAPLN1, and cell proliferation is promoted, eventually leading to hair growth.

Therefore, the HAPLN1 protein of the present invention promotes cell proliferation through Ras-ERK1/2 signaling activated by TGF-β protein, and can be used for preventing or treating hair loss. In particular, the HAPLN1 protein of the present invention promotes the proliferation of hair germinal matrix cells, thereby inducing hair growth.

As used herein, the term "hair loss" refers to a state in which there is no hair in the area where the hair should normally exist, regardless of the cause thereof, and may be, for example, male-pattern hair loss, female-pattern hair loss, circular hair loss, or telogen hair loss.

In another embodiment, the present invention provides a cosmetic composition for preventing or improving hair loss, comprising HAPLN1 as an active ingredient. The cosmetic composition may be, for example, a cosmetic for hair, and the formulation thereof is not particularly limited, and may be appropriately selected depending on the purpose intended.

For example, the cosmetic composition may be formulated as a solution, suspension, emulsion, paste, gel, cream, lotion, powder, soap, surfactant-containing cleansing, oil, powder foundation, emulsion foundation, wax foundation, and spray, but is not limited thereto. More specifically, the cosmetic composition for hair may include, for example, a cleaning agent, such as shampoo, conditioner, and body cleanser, a hairdressing agent, such as hair tonic, gel or mousse, a hair nourishing agent, or a hair dye.

In one embodiment of the present invention, the cosmetic composition may contain various suitable bases and additives, as necessary, and the types and amounts of these ingredients can be easily selected by the inventor. The cosmetic composition may contain acceptable additives, as necessary, and may further include, for example, components, such as preservatives, colorants, additives, etc. that are commonly used in the art.

### MODE OF DISCLOSURE

Hereinafter, the present invention will be described in more detail through the following examples, but the following examples are for illustrative purposes only and are not intended to limit the scope of the present invention.

### [Example 1]

### Confirmation of HAPLN1 Protein and HAPLN1 mRNA Expression in each Hair Growth Cycle

In this example, the expression of HAPLN1 protein was confirmed in each hair growth cycle using mouse skin.

The mouse skin was collected at 23 days old (early anagen), 32 days old (anagen), 40 days old (catagen), and 44 days old (telogen), and was fresh frozen. The skin tissue section was fabricated to have a thickness of 8 µm, and the presence or absence of the HAPLN1 protein was detected using a HAPLN1 antibody (Abcam, USA). Immunofluorescence was carried out according to a common experimental method.

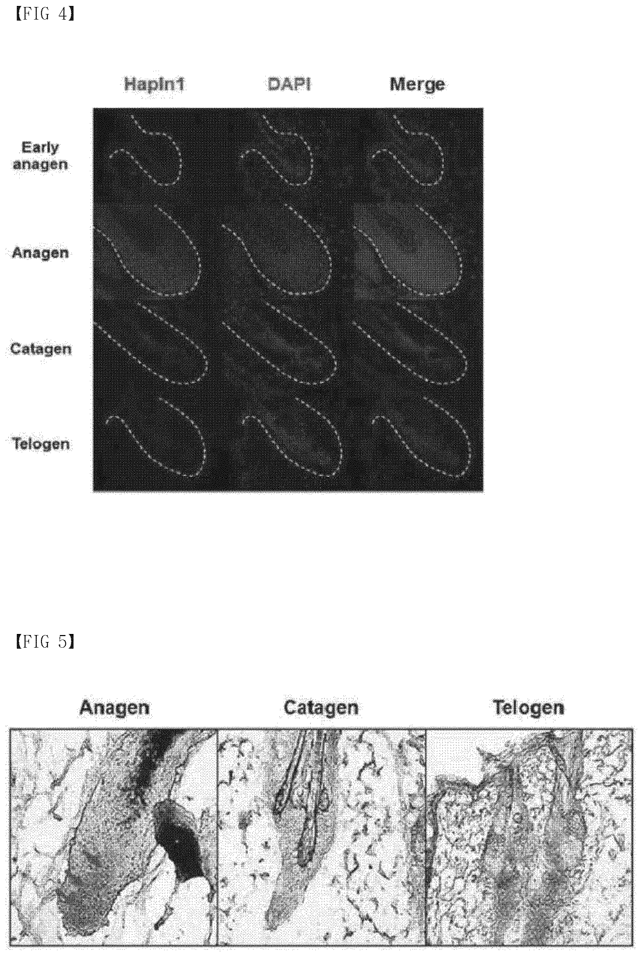

As a result, as shown in FIG. 4, it was confirmed that HAPLN1 was significantly expressed in the hair germinal matrix cells in the anagen phase, and it was also confirmed that the HAPLN1 expression level was reduced in the catagen and telogen phases.

Subsequently, HAPLN1 mRNA was identified in the mouse skin to determine in which cells HAPLN1 was produced.

The mouse skin was collected at 32 days old (anagen), 40 days old (catagen), and 44 days old (telogen), and the skin tissue section was paraffin-sliced to a thickness of 8 µm. Detection was performed by using a HAPLN1 mRNA probe (ACDbio, USA), and in-situ hybridization experimentation was performed according to the manufacturer's experimental method (ACDbio; RNAscope^{®} 2.5 HD Assay-BROWN, CA, USA).

As a result, as shown in FIG. 5, HAPLN1 mRNA was confirmed to be expressed from the hair germinal matrix cells in the anagen, and the expression was not confirmed in the catagen and telogen phases.

### [Example 2]

### Confirmation of Increase in TβRII Protein in Human Hair Germinal Matrix Cells by HAPLN1

In this example, it was confirmed whether HAPLN1 increased the amount by preventing the degradation of TβRII.

HAPLN1 was treated on human hair germinal matrix cells by concentration. Specifically, human hair germinal matrix cells (HHGMC) were dispensed into a poly-D-lysine 6-well plate at 5.0 × 10⁴ per well and cultured for 24 hours. After 24 hours, the medium was removed and replaced with a new serum-free medium. HAPLN1 was treated with 0, 5, 10, 20 ng/mL and incubated for 24 hours. After collecting cells, a lysis buffer (25 mM Tris-HCI, 1 mM EDTA, 0.1% Triton-X100, phosphatase inhibitor, and protease inhibitor) was added, and all the cells were broken through sonication. TβRII in the sample was measured using Western blotting. The optical density of TβRII was compared to that of GAPDH using a densitometer (that is, TβRII optical concentration ÷ GAPDH optical concentration). Here, GAPDH was used as a loading control.

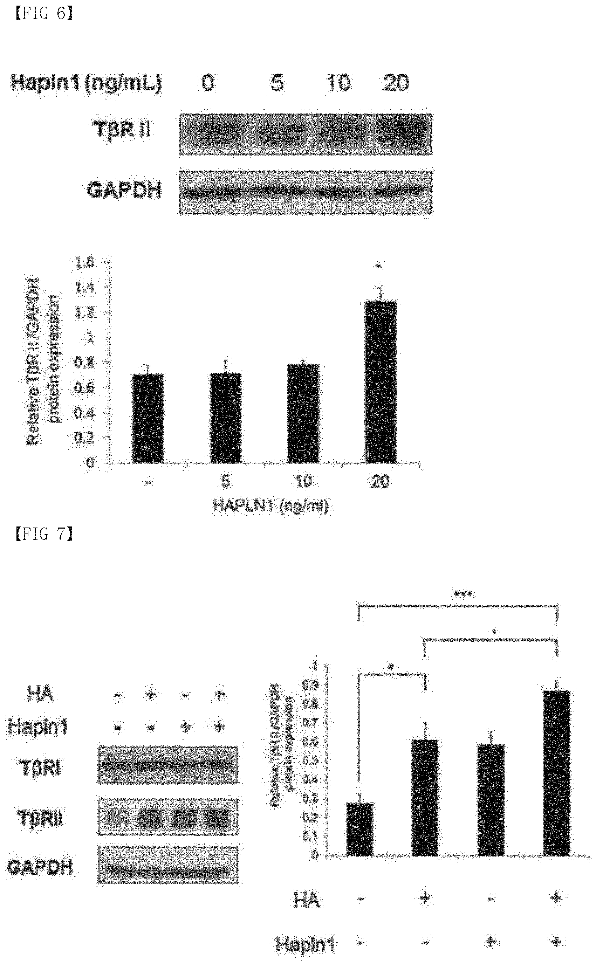

As a result, as shown in FIG. 6, it was confirmed that TβRII was increased at 20 ng/mL of HAPLN1.

From this, it was confirmed that TβRII was increased when HAPLN1 was treated on human hair germinal matrix cells.

### [Example 3]

### Confirmation of Increase in TβRII Protein in Human Hair Germinal Matrix Cells by HAPLN1 and/or HA

It was assumed that HAPLN1 affects TβRII by stabilizing HA rather than acting directly on TβRII in the TGF-β signaling pathway. To confirm this, an experiment for treating HAPLN1 and HA together was performed.

Human hair germinal matrix cells were dispensed into a poly-D-lysine 6-well plate at a concentration of 5.0 × 10⁴ per well and cultured for 24 hours. After 24 hours, the medium was removed and replaced with a new serum-free medium. HAPLN1 was treated with 25 ng/mL, HA was treated with 25 µg/mL, and after 1 hour, TGF-β2 (2 ng/mL) was treated. After 23 hours, cells were collected, a lysis buffer (25 mM Tris-HCI, 1 mM EDTA, 0.1% Triton-X100, phosphatase inhibitor, and protease inhibitor) was added, and all the cells were broken through sonication. TβRI and TβRII in the sample were measured using Western blotting. The optical density of TβRII was compared to that of GAPDH using a densitometer.

As a result, as shown in FIG. 7, it was confirmed that TβRII was increased when HAPLN1 or HA was treated on the human hair germinal matrix cells.

From this, it was reconfirmed that the TβRII was significantly increased when HAPLN1 was treated alone, and, in particular, it was confirmed that HAPLN1 further enhanced the increase of TβRII by HA. However, since there was no change in the concentration of TβRI, the increase of TβRII by HAPLN1 is considered to be selective.

### [Example 4]

### Confirmation of Effect of Endogenous HAPLN1 Deficiency on TβRII Protein Concentration in Human Hair Germinal Matrix Cells

In order to confirm the effect of endogenous HAPLN1 on the TβRII protein concentration in human hair germinal matrix cells, which were made to be deficient in endogenous HAPLN1 by using HAPLN1 siRNA.

Human hair germinal matrix cells were dispensed into a poly-D-lysine 6-well plate at a concentration of 5.0 × 10⁴ per well and cultured for 24 hours. After 24 hours, the medium was removed and replaced with a low-serum medium. HAPLN1 siRNA and scrambled siRNA were added in each amount of 25 pmol per well and then cultured for 24 hours. After collecting cells, a lysis buffer (25 mM Tris-HCI, 1 mM EDTA, 0.1% Triton-X100, phosphatase inhibitor, and protease inhibitor) was added, and all the cells were broken through sonication. HAPLN1 and TβRII in the sample were measured using Western blotting. The optical density of each of HAPLN1 and TβRII was compared to that of GAPDH using a densitometer.

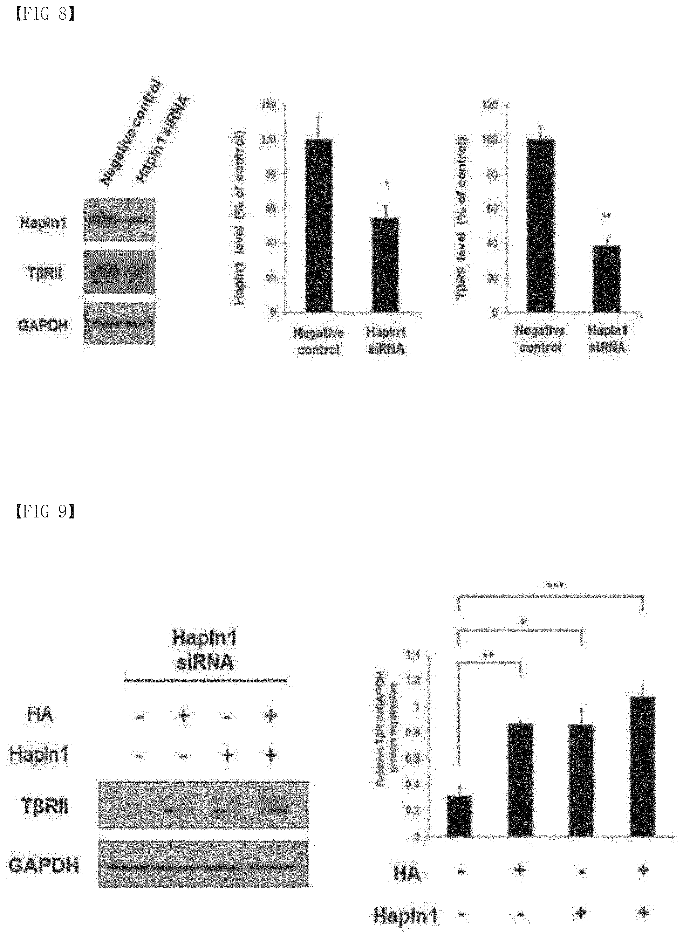

As a result, as shown in FIG. 8, it was confirmed that TβRII was also decreased when the human hair germinal matrix cells, which were made to be deficient in endogenous HAPLN1 by treating HAPLN1 siRNA.

### [Example 5]

### Confirmation of Effect of Exogenous HAPLN1 and/or HA on TβRII Protein Concentration in Endogenous HAPLN1-Deficient Human Hair Germinal Matrix Cells

It was determined whether the TβRII protein was increased in human hair germinal matrix cells, which were made to be deficient in HAPLN1 by using HAPLN1 siRNA.

The human hair germinal matrix cells were dispensed into a poly-D-lysine 6-well plate at a concentration of 5.0 × 10⁴ per well and cultured for 24 hours. After 24 hours, the medium was removed and replaced with a low-serum medium. HAPLN1 siRNA and scrambled siRNA were added at a concentration of 25 pmol per well and incubated for 24 hours. After the siRNA and the medium were all removed, the medium was replaced with a serum-free medium having HAPLN1 (25 ng/mL) or HA (25 µg/mL) added thereto, and cultured for 1 hour. TGF-β2 (2 ng/mL) was treated and incubated for 23 hours. After collecting cells, a lysis buffer (25 mM Tris-HCI, 1 mM EDTA, 0.1% Triton-X100, phosphatase inhibitor, and protease inhibitor) was added, and all the cells were broken through sonication. TβRII in the sample was measured using Western blotting. The optical density of TβRII was compared to that of GAPDH using a densitometer.

As a result, as shown in FIG. 9, it was confirmed that decreased TβRII was recovered when the exogenous HAPLN1 and/or HA was treated on the human hair germinal matrix cells deficient in the endogenous HAPLN1.

### [Example 6]

### Confirmation of Effect of Exogenous HAPLN1 on Concentrations of TβRII and HAS2 Proteins in Human Hair Germinal Matrix Cells Deficient in Endogenous HA

4-MU (4-methylumbelliferone) is an inhibitor of HA-producing hyaluronan synthase 2 (HAS2), which produces HA. In order to confirm the effect of HA produced in cells, HA production in cells was inhibited using 4-MU.

The human hair germinal matrix cells, in which the endogenous HA was inhibited by treating 4-MU, were dispensed into a poly-D-lysine 6-well plate at a concentration of 5.0 × 10⁴ per well and cultured for 24 hours. After 24 hours, the medium was removed and replaced with a new serum-free medium. HAPLN1 was treated with 25 ng/mL, and after 1 hour, TGF-β2 (2 ng/mL) was treated. After 23 hours, cells were collected, a lysis buffer (25 mM Tris-HCI, 1 mM EDTA, 0.1% Triton-X100, phosphatase inhibitor, and protease inhibitor) was added, and all the cells were broken through sonication. HAS2 and TβRII in the sample were measured using Western blotting. The optical density of each of HAS2 and TβRII was compared to that of GAPDH using a densitometer.

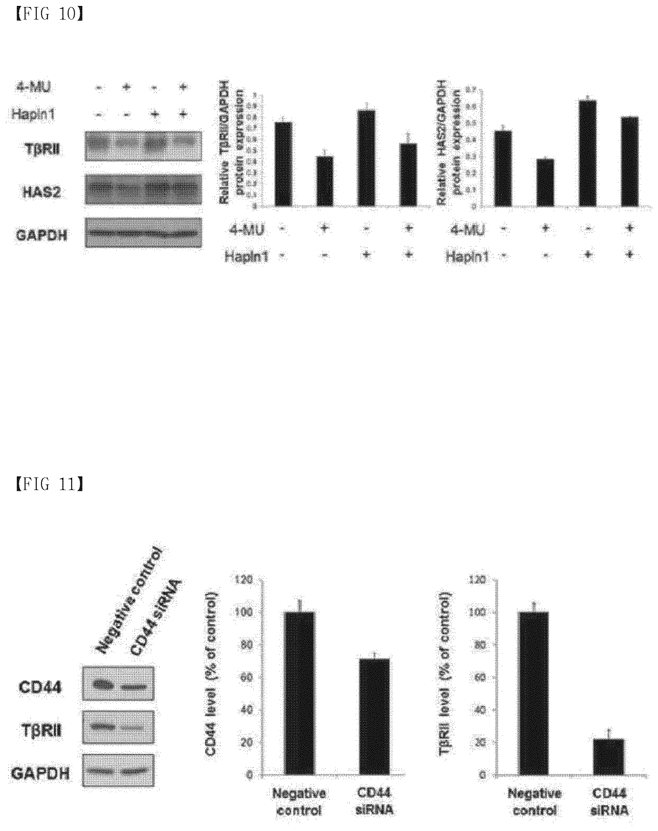

As a result, as shown in FIG. 10, it was confirmed that HAS2 and TβRII levels were decreased when treated with 4-MU, and HAS2 and TβRII levels were recovered when HAPLN1 was treated.

### [Example 7]

### In Example 3, it was confirmed that HAPLN1 regulates TβRII through HA. However, HA does not directly bind to TβRII, but CD44, which is a receptor for HA, binds to TβRII.

In this example, it was confirmed whether CD44 was one of the important factors in the TβRII regulation process, and whether CD44 was an actually essential factor in the process of increasing TβRII by HAPLN1.

### 1. Confirmation of Effect of CD44 Deficiency on Concentration of TβRII Protein in Human Hair Germinal Matrix Cells

First, it was confirmed how the concentration of TβRII protein changed when human hair germinal matrix cells were deficient in CD44.

The human hair germinal matrix cells, which were made to be deficient in endogenous CD44 by treating CD44 siRNA, were dispensed into a poly-D-lysine 6-well plate at a concentration of 5.0 × 10⁴ per well and cultured for 24 hours. After 24 hours, the medium was removed and replaced with a low-serum medium. CD44 siRNA and scrambled siRNA were added at a concentration of 25 pmol per well and incubated for 24 hours. After collecting cells, a lysis buffer (25 mM Tris-HCI, 1 mM EDTA, 0.1% Triton-X100, phosphatase inhibitor, and protease inhibitor) was added, and all the cells were broken through sonication. CD44 and TβRII in the sample were measured using Western blotting. The optical density of each of CD44 and TβRII was compared to that of GAPDH using a densitometer.

As a result, as shown in FIG. 11, it was confirmed that the concentration of TβRII protein was significantly reduced in human hair germinal matrix cells deficient in CD44.

### 2. Confirmation of Effect(s) of HAPLN1 and/or HA on Concentration of TβRII Protein in Human Hair Germinal Matrix Cells Deficient in CD44

Subsequently, the effect(s) of HAPLN1 and/or HA on the concentration of TβRII protein in CD44-deficient human hair germinal matrix cells was confirmed.

The human hair germinal matrix cells, which were made to be deficient in CD44 by using CD44 siRNA, were dispensed into a poly-D-lysine 6-well plate at a concentration of 5.0 × 10⁴ per well and cultured for 24 hours. After 24 hours, the medium was removed and replaced with a low-serum medium. CD44 siRNA and scrambled siRNA were added at a concentration of 25 pmol per well and incubated for 24 hours. After the siRNA and the medium were all removed, the medium was replaced with a serum-free medium having HAPLN1 (25 ng/mL) or HA (25 µg/mL) added thereto, and cultured for 1 hour. TGF-β2 (2 ng/mL) was treated and incubated for 23 hours. After collecting cells, a lysis buffer (25 mM Tris-HCI, 1 mM EDTA, 0.1% Triton-X100, phosphatase inhibitor, and protease inhibitor) was added, and all the cells were broken through sonication. TβRII in the sample was measured using Western blotting. The optical density of TβRII was compared to that of GAPDH using a densitometer.

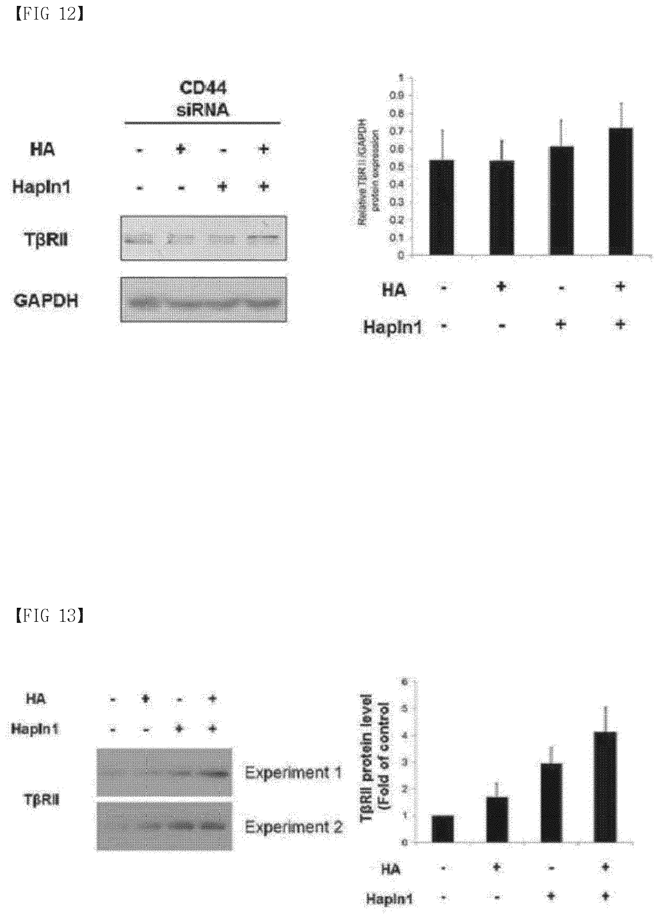

As a result, as shown in FIG. 12, it was not confirmed that TβRII was recovered even with HAPLN1 and/or HA treatment when the human hair germinal matrix cells were deficient in CD44.

From the above experiment, it was confirmed that CD44 was an essential factor in the process of increasing TβRII by HAPLN1 and HA.

### [Example 8]

### Confirmation of Effect(s) of HAPLN1 and/or HA on Concentration of Cell Membrane TβRII Protein

It was assumed that HAPLN1 would bring about an increase in cell membrane TβRII by inhibiting the endocytosis of TβRII, and an experiment to prove this was performed.

All proteins present in the cell membrane were labeled with biotin, and immunoprecipitation was performed with a biotin antibody to isolate only the cell membrane protein. In order to confirm changes in the ratio of TβRII among cell membrane proteins, Western blotting was performed. The specific experimental method will now be described.

Human hair germinal matrix cells were dispensed into a poly-D-lysine 100 mm dish at a concentration of 2.7 × 10⁶ and cultured for 24 hours. After 24 hours, the medium was removed and replaced with a new serum-free medium. HAPLN1 was treated with 25 ng/mL and HA with 25 µg/mL, and after 1 hour, TGF-β2 (2 ng/mL) was treated. To label biotin after 23 hours, EZ-Link^{™} Sulfo-NHS-LC-Biotin (Thermo Fisher Scientific, MA, USA) was treated at a concentration of 250 µg/mL and incubated at 4 °C for 1 hour. The biotin labeling reaction was terminated by treatment with 50 mM Tris-HCI. After collecting cells, a lysis buffer (50 mM Tris-HCI, 150 mM NaCl, 1% NP-40, phosphatase inhibitor, and protease inhibitor) was added, and all the cells were broken through sonication. Cell membrane proteins were isolated by performing immunoprecipitation experiments using anti-biotin antibodies. TβRII in the isolated membrane protein sample was measured using Western blotting. The optical density of TβRII was compared to that of GAPDH using a densitometer.

As a result, as shown in FIG. 13, it was confirmed that the cell membrane TβRII protein concentration was increased when treated with HAPLN1 and/or HA.

### [Example 9]

### Investigation of Cell Proliferation Effect and Action Mechanism of HAPLN1

In this example, in order to confirm how HAPLN1 induces cell proliferation, the effect of HAPLN1 on the Smad2 pathway and ERK1/2 pathway was determined.

Human hair germinal matrix cells were dispensed into a poly-D-lysine 6-well plate at a concentration of 5.0 × 10⁴ per well and cultured for 24 hours. After 24 hours, the medium was removed and replaced with a new serum-free medium. HAPLN1 was treated with 25 ng/mL and HA with 25 µg/mL, incubated for 23 hours, and then stimulated with TGF-β2 (2 ng/mL) for 1 hour. After collecting cells, a lysis buffer (25 mM Tris-HCI, 1 mM EDTA, 0.1% Triton-X100, phosphatase inhibitor, and protease inhibitor) was added, and all the cells were broken through sonication. p-ERK1/2, p-Smad2, p-MEK1/2, and p-c-Raf in the sample were measured using Western blotting. Optical densities of p-ERK1/2, p-Smad2, p-MEK1/2, and p-c-Raf were compared to those of ERK1/2, Smad2/3, MEK1, and c-Raf using a densitometer. Here, ERK1/2, Smad2/3, MEK1, and c-Raf were used as loading controls.

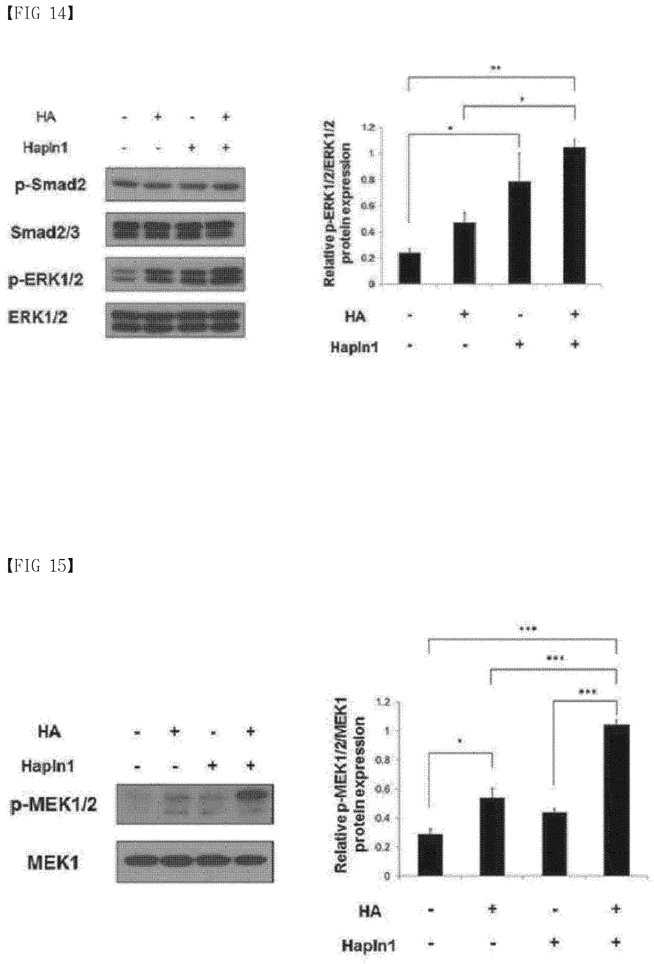

As a result, as shown in FIGS. 14 to 16, it was confirmed that ERK1/2 signaling was activated by TGF-β2 in cells treated with HAPLN1 and/or HA. In addition, it was confirmed that HAPLN1 enhanced the activation of ERK1/2 signaling of HA. However, there was no change in the phosphorylation of Smad2. In addition, it was confirmed that MEK1/2 and c-Raf, which are in the upstream mechanisms of ERK1/2, had increased activities by HAPLN1 and/or HA treatment.

From this, it was confirmed that the ERK signal was activated by HAPLN1, indicating that signaling is achieved through a non-canonical pathway. However, it was confirmed that HAPLN1 did not activate the canonical pathway of Smad2/3.

### [Example 10]

### Investigation of Cell Proliferation Effect and Action Mechanism of HAPLN1

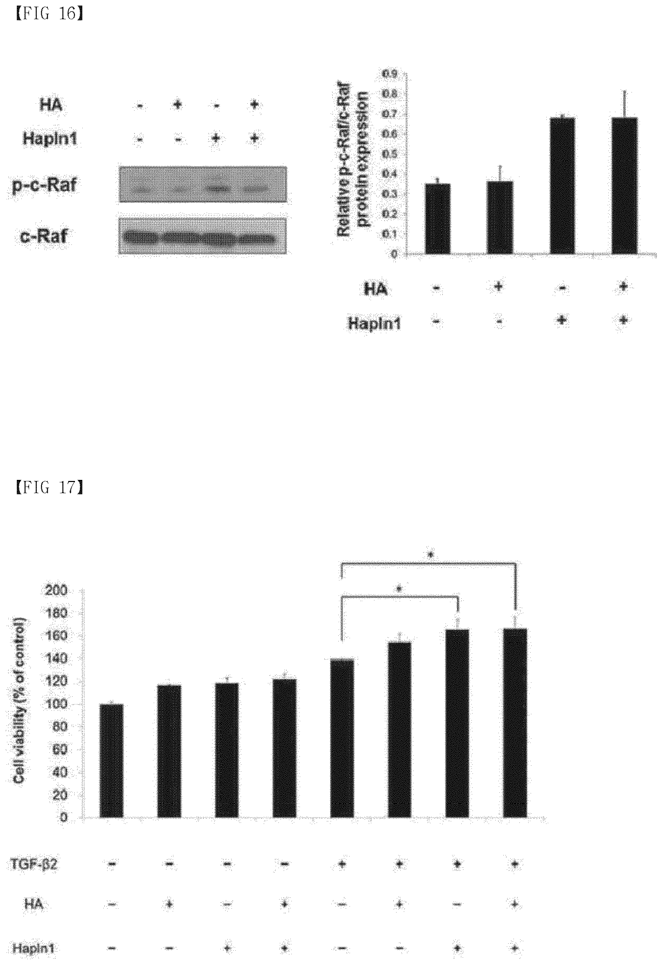

Human hair germinal matrix cells were dispensed into a poly-D-lysine 96-well plate at a concentration of 2.0 × 10⁴ per well and cultured for 24 hours. After 24 hours, the medium was removed and replaced with a new serum-free medium. HAPLN1 was treated with 25 ng/mL and HA with 25 µg/mL, incubated for 1 hour, and then stimulated with TGF-β2 (2 ng/mL) for 23 hours. CCK-8 (Enzo Biochem, NY, USA) was treated and incubated at 37 °C for 1 hour. Absorbance was measured at 450 nm, and the results are shown in FIG. 17.

It was confirmed that cell proliferation was promoted in the group treated with HAPLN1 and/or HA in the presence of TGF-β2, compared to the control group. This suggests that HAPLN1 promotes the proliferation of human hair germinal matrix cells through the non-canonical TGF-β signaling pathway.

### [Example 11]

### Confirmation of Expression of HAPLN1 Protein and TβRII Protein in each Hair Growth Cycle

In this example, HAPLN1 and TβRII were fluorescently stained in each hair cycle of the mouse skin, and the expression of HAPLN1 protein and TβRII protein was confirmed.

The mouse skin was collected at 32 days old (anagen), 40 days old (catagen), and 44 days old (telogen), and was fresh frozen. The skin tissue section was fabricated to have a thickness of 8 µm, and the presence or absence of the HAPLN1 and TβRII proteins was detected using a HAPLN1 antibody (Abcam, USA) and a TβRII antibody. Immunofluorescence was carried out according to a common experimental method.

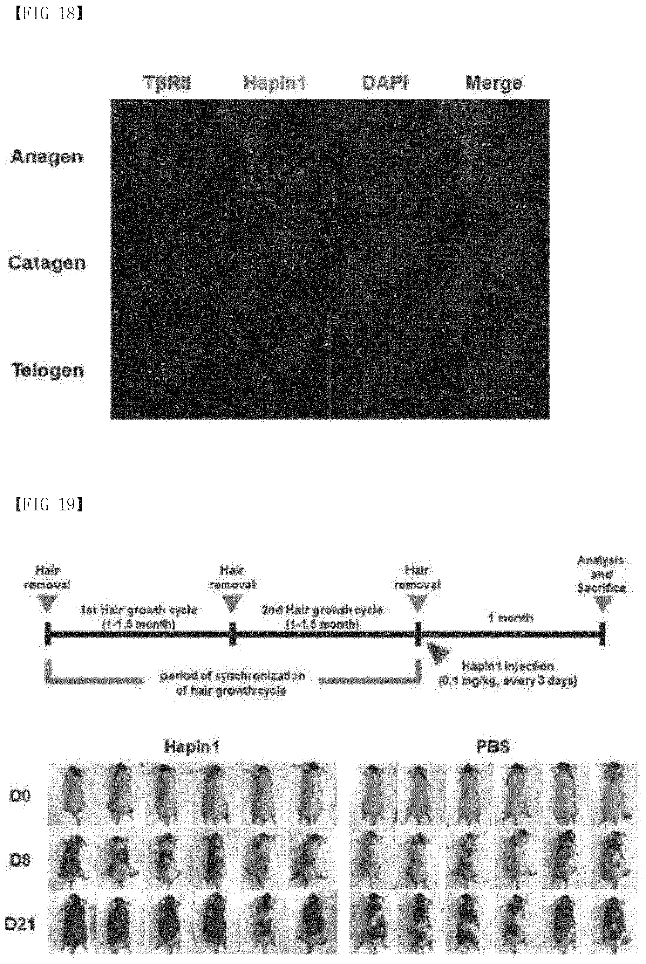

As a result, as shown in FIG. 18, it was confirmed that HAPLN1 was expressed in the hair germinal matrix cells in the anagen phase, and it was also confirmed that the expression of HAPLN1 was reduced in the catagen and telogen phases. In addition, it was confirmed that HAPLN1 and TβRII were expressed at the same location in the hair germinal matrix of the anagen phase (co-localization). This suggests that HAPLN1 and TβRII contribute to the proliferation of human hair germinal matrix cells.

### [Example 12]

### Observation of Changes in Hair Growth Cycle by HAPLN1 in Animal Models

### 1. Confirmation of Effect of Intraperitoneal Systemic Administration of HAPLN1 on Mouse Hair Growth

HAPLN1 in the body decreases with age. HAPLN1 was administered to 20-month-old C57 mice with a decrease in HAPLN1 in the body.

Since C57 mice had different hair growth cycles, the cycles of all mice were uniformly adjusted through two hair growth cycles (see the schedule of FIG. 19). Thereafter, HAPLN1 was injected intraperitoneally at 0.1 mg/kg once every 3 days.

As a result, as shown in FIG. 19, the group treated with HAPLN1 entered the anagen phase in a short period of time.

2. Confirmation of Effect of Intraperitoneal Systemic Administration of HAPLN1 siRNA on Mouse Hair Growth

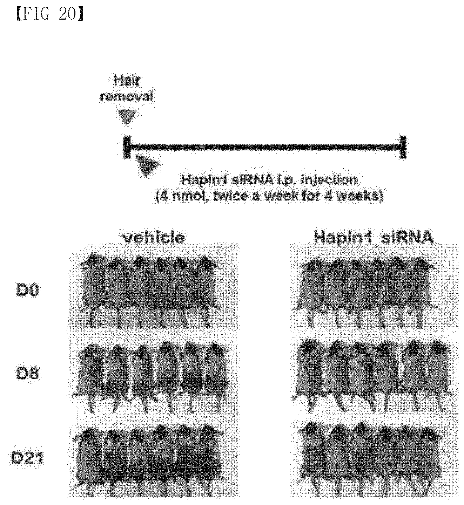

HAPLN1 siRNA was administered to 7-week old C57 mice, and how the hair growth cycle changed according to HAPLN1 deficiency was observed. To this end, HAPLN1 siRNA (Dharmacon; Accell HAPLN1 siRNA SMARTpool, CO, USA) was intraperitoneally injected twice a week at 4 nmol for 4 weeks (see the schedule of FIG. 20).

As a result, as shown in FIG. 20, it was confirmed that the group to which HAPLN1 siRNA was administered showed relatively slow entry into the hair growth cycle, compared to the control group.

### [Example 13]

### Verification of Effect of HAPLN1, CX3CL1 and CDON in Proliferating Dermal Papillary Cells or Hair Germinal Matrix Cells

U.S. Patent No. 8,334,136 proposes a possibility that hair follicle formation or hair regeneration can be promoted by maintaining or increasing the expression of cell adhesion genes, such as HAPLN1, CX3CL1, CDON, etc. present in dermal papilla cells. In this example, when the HAPLN1, CX3CL1 or CDON protein expressed by the cell adhesion genes was directly treated on dermal papilla cells or hair germinal matrix cells, it was verified whether or not the proliferative effect was actually expressed.

### 1. Confirmation of Dermal Papilla Cell Proliferating Effect

Human dermal papilla cells were cultured in a 37°C, 5% CO2 incubator using a dermal papilla cell proliferation medium (Promocell). When the cells were about 90% full, the cells were detached with a 0.05% Trypsin/EDTA solution and then centrifuged at 1000 rpm for 3 minutes to recover only the cells. The cells were dispensed into a 96-well plate at 2.0 × 103 per well, cultured for 24 hours, and HAPLN1, CX3CL1 and CDON were diluted to have a final concentration of 25 ng/ml in serum-free medium. After removing the existing culture medium, 200 µl of diluted HAPLN1, CX3CL1, and CDON were dispensed into each well and cultured for 1 hour. To reduce an experimental variation, 3 wells per group were treated (triplication). In addition, minoxidil, which is known to proliferate dermal papilla cells, was treated with 10 µM and set as a positive control.

In the same manner as described above, HAPLN1 (25 ng/ml), CX3CL1 (25 ng/ml), CDON (25 ng/ml) or minoxidil (10 µM), and human recombinant TGF-β2 were diluted to a final concentration of 2 ng/ml, and the medium of the group containing TGF-β2 was replaced by 200 µl. To reduce an experimental variation, 3 wells per group were treated (triplication).

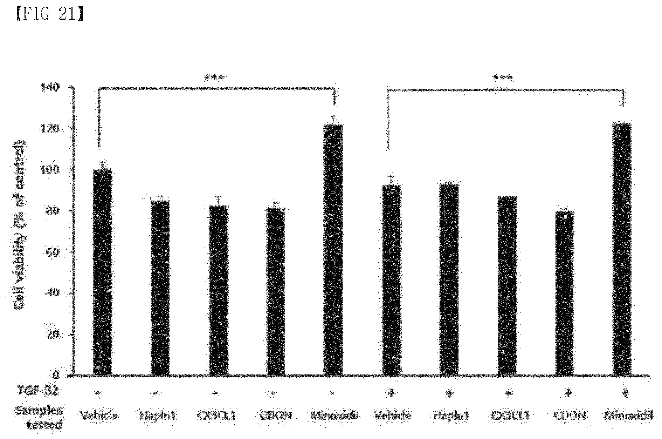

After incubation for 23 hours, the plate was removed from the incubator, and 20 µl of CCK-8 (WST-8) solution was added to the plate containing the medium and the reagent per 200 µl of the medium for the reaction to be carried out at 37 °C for 1 hour. Then, absorbance was measured at 450 nm using a microplate reader, and the results thereof are shown in FIG. 21.

As shown in FIG. 21, when TGF-β2 is present or absent, significant dermal papilla cell proliferating activity was confirmed in the positive control group treated with minoxidil (***P<0.001). However, All of HAPLN1, CX3CL1 and CDON did not proliferate dermal papilla cells or rather inhibited proliferation thereof. That is, it can be seen that even if the protein expressed by the cell adhesion genes in the dermal papilla cells is administered to the dermal papilla cells, the proliferation effect of the dermal papilla cells does not appear.

### 2. Confirmation of Hair Germinal Matrix Cell Proliferating Effect

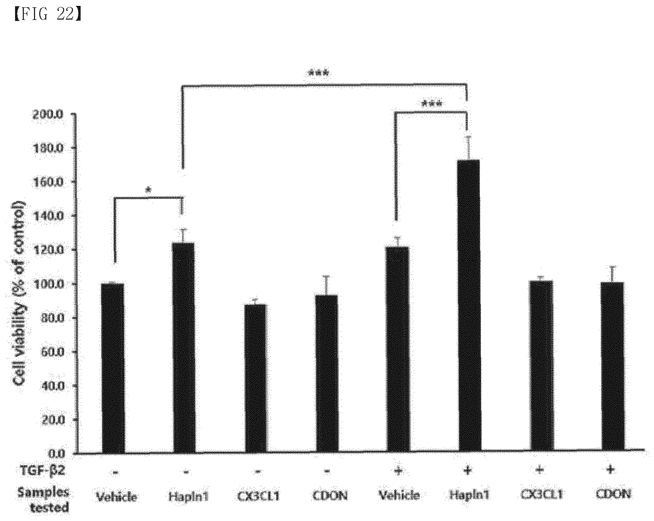

Human hair germinal matrix cells were released in a poly-D-lysine-coated flask using a mesenchymal stem cell medium (MSCM), and cultured in a 5% CO2 incubator at 37 °C. When the cells were about 90% full, the cells were detached with a 0.05% Trypsin/EDTA solution, and then only the cells were recovered by centrifugation at 1000 rpm for 3 minutes. The recovered cells were dispensed into 96-well poly-D-lysine coated plates (BD bioscience) at 2.0 × 103 per well and cultured for 24 hours, and the final concentrations of HAPLN1, CX3CL1 and CDON were diluted to 25 ng/ml in a serum-free medium. The subsequent process was performed in the same manner as the experiment for the dermal papilla cells to measure the absorbance, and the results are shown in FIG. 22.

As shown in FIG. 22, HAPLN1 showed significant hair germinal matrix cell proliferating activity in the absence of TGF-β2 (*P<0.05), and, particularly superior hair germinal matrix cell proliferating activity in the presence of TGF-β2 (***P< 0.001). Therefore, it was confirmed to have a proliferating effect of human hair germinal matrix cells through the TGF-β signaling pathway. However, like in the dermal papilla cells, CX3CL1 and CDON showed a result of inhibiting cell proliferation in hair germinal matrix cells as well.

Although U.S. Patent No. 8,334,136 states a possibility that hair follicle formation or hair regeneration can be promoted by maintaining or increasing the expression of cell adhesion genes present in dermal papilla cells, it can be eventually confirmed that that all proteins expressed by cell adhesion genes do not actually proliferate dermal papilla cells or hair germinal matrix cells.

Therefore, it can be seen that only the HAPLN1 protein has the effect of preventing or treating hair loss by promoting hair germinal matrix cell proliferation and hair growth.

While specific parts of the present invention have been described in detail, it is obvious to a person skilled in the art that such specific description is only a preferred embodiment, and the scope of the present invention is not limited thereby. Therefore, the practical scope of the present invention will be defined by the appended claims and equivalents thereof.

## Claims

1. A pharmaceutical composition for preventing or treating hair loss, comprising hyaluronan and proteoglycan link protein 1 (HAPLN1) as an active ingredient.

2. The pharmaceutical composition of claim 1, wherein the HAPLN1 activates a non-canonical signaling pathway, and the non-canonical signaling pathway is an ERK1/2 signaling pathway activated by a TGF-β protein.

3. The pharmaceutical composition of claim 1, wherein the HAPLN1 causes hair growth by promoting proliferation of hair germinal matrix cells.

4. The pharmaceutical composition of claim 1, wherein the hair loss is male-pattern hair loss, female-pattern hair loss, alopecia areata, or telogen hair loss.

5. A cosmetic composition for preventing or improving hair loss, comprising hyaluronan and proteoglycan link protein 1 (HAPLN1) as an active ingredient.

6. The cosmetic composition of claim 5, wherein the HAPLN1 activates a non-canonical signaling pathway, and the non-canonical signaling pathway is an ERK1/2 signaling pathway activated by a TGF-β protein.

7. The cosmetic composition of claim 5, wherein the HAPLN1 causes hair growth by promoting proliferation of hair germinal matrix cells.
